(19) Europäisches Patentamt
European Patent Office
Office européen des brevets

(11) EP 0 583 338 B1

(12) **FASCICULE DE BREVET EUROPEEN**

(45) Date de publication et mention de la délivrance du brevet: **12.03.1997 Bulletin 1997/11**

(21) Numéro de dépôt: **92910222.6**

(22) Date de dépôt: **22.04.1992**

(51) Int Cl.[6]: **C07K 1/06**, C07D 233/64, C07C 279/24, C07C 271/22, C07C 239/06

(86) Numéro de dépôt international: **PCT/FR92/00361**

**WO 92/19643 (12.11.1992 Gazette 1992/28)**

(54) **PROCEDE DE SYNTHESE DE PEPTIDES, NOUVEAUX DERIVES D'ACIDES AMINES MIS EN OEUVRE DANS CE PROCEDE ET LEURS PROCEDES DE PREPARATION**

Verfahren zur Synthese von Peptiden, Derivate von Aminosäuren zur Verwendung bei dem obengenannten Verfahren und Methoden zu deren Herstellung.

METHOD FOR SYNTHESIZING PEPTIDES, NOVEL AMINO ACID DERIVATIVES USED THEREIN, AND METHODS FOR PREPARING SAME

(84) Etats contractants désignés: **BE CH DE DK FR GB IT LI NL SE**

(30) Priorité: **25.04.1991 FR 9105093**

(43) Date de publication de la demande: **23.02.1994 Bulletin 1994/08**

(73) Titulaire: **PROPEPTIDE**
**91710 Vert-le-Petit (FR)**

(72) Inventeurs:
- **LOFFET, Albert**
  **F-75015 Paris (FR)**
- **ZHANG, Hai**
  **F-75015 Paris (FR)**

(74) Mandataire: **Pech, Bernard et al**
**SNPE - Service Propriété Industrielle**
**12, Quai Henri IV**
**75181 Paris Cédex 04 (FR)**

(56) Documents cités:
**EP-A- 0 401 817** **WO-A-91/01331**

- **JOURNAL OF MEDICINAL CHEMISTRY. vol. 33, no. 6, 1 Juin 1990, WASHINGTON US pages 1620 - 1634; G TOUS ET AL.: '0-(epoxyalkyl)tyrosines and**
- **(epoxyalkyl)phenylalanine as irreversible inactivators of serine proteases: synthesis and inhibition mechanism'**
- **JOURNAL OF ORGANIC CHEMISTRY. vol. 52, no. 22, 30 Septembre 1987, EASTON US pages 4984 - 4993; O DANGLES ET AL.: 'selective cleavage of the allyl and allyloxycarbonyl groups through palladium-catalyzed hydrostannolysis with tributyltin hydride. Application to the selective protection-deprotection of aminoacid derivatives and in peptide synthesis'**
- **TETRAHEDRON LETTERS. vol. 28, no. 24, 1987, OXFORD GB pages 2737 - 2740; I MINAMI ET AL.: '1-isoproplylallyloxycarbonyl (Ipaoc) as a protective group of amines and its deprotection catalised by palladium-phosphine complex'**
- **JOURNAL OF THE AMERICAN CHEMICAL SOCIETY. vol. 72, no. 2, 28 Février 1950, GASTON, PA US pages 725 - 727; C M STEVENS ET AL: 'amino acid derivatives. I. Carboallyloxy derivatives of alpha aminoacids'**
- **TETRAHEDRON LETTERS. vol. 27, no. 21, 1986, OXFORD GB pages 2365 - 2368; F GUIBE ET AL.: 'Palladium-catalyzed reaction of tributyltin hydride. Selective and very mild deprotection of allyl and allyloxycarbonyl derivatives of**
- **aminoacids'**
- **CHEMICAL ABSTRACTS, vol. 104, no. 21, 26 Mai 1986, Columbus, Ohio, US; abstract no. 186834U, H KUNZ ET AL.: 'Allyl ester as temporary protecting group for the beta-carboxy function of aspartic acid' page 676 ;**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 583 338 B1

- CHEMICAL ABSTRACTS, vol. 114, no. 11, 12 Mars 1990, Columbus, Ohio, US; abstract no. 102741G, P J BELSHAW ET AL.: 'Chlorotrimethysilane mediated formation of omega-allyl esters of aspartic and glutamic acids' page 822 ;
- CHEMICAL ABSTRACTS, vol. 114, no. 11, 12 Mars 1990, Columbus, Ohio, US; abstract no. 102744K, A SCHOENFELDER ET AL.: 'convenient preparation of dissymmetrical diesters of n-boc glutamic acid' page 822 ;
- PEPTIDES: CHEMISTRY AND BIOLOGY. PROCEEDINGS OF THE 12TH AMERICAN PEPTIDE SYMPOSIUM ( J A SMITH AND J RIVIER, EDS) 1992, ESCOM, LEIDEN pages 583 - 584; M H LYTTLE AND D HUDSON: 'ALLYL-BASED SIDE CHAIN PROTECTION FOR spps'

## Description

L'invention concerne un nouveau procédé de synthèse de peptides, les nouveaux dérivés d'acides aminés mis en oeuvre dans ce procédé et leurs procédés de préparation.

Les peptides sont des enchaînements d'acides aminés. Ils sont obtenus dans la plupart des cas au moyen de la condensation avec élimination d'une molécule d'eau d'un groupe carboxyle d'un acide aminé avec le groupe amine d'un autre acide aminé. L'enchaînement peut être effectué en additionnant un autre acide aminé à l'enchaînement précédemment obtenu ou en condensant des fragments de peptides préalablement formés. Pendant toutes ces condensations les groupes amines et carboxyles qui ne participent pas à la réaction sont en général bloqués par des groupes protecteurs qui doivent être fixés facilement, être stables pendant la réaction de condensation et être éliminés de façon sélective l'un par rapport à l'autre. Si les acides aminés qui constituent le peptide ont des chaînes latérales qui contiennent des groupes fonctionnels réactifs tels que des groupes amino, carboxy, hydroxy, thiol, le problème de la protection devient très difficile à résoudre car il faut que les groupes protecteurs de ces groupes fonctionnels soient stables non seulement pendant l'opération de condensation mais également pendant les étapes de déprotection du groupe amino en $\alpha$ d'une part et du groupe carboxyle en a d'autre part. Il faut également qu'ils puissent être éliminés à la fin du procédé et que l'on puisse récupérer le peptide intact. De nombreux groupes protecteurs ont été proposés mais on est confronté en particulier pour la synthèse de peptides à longue chaîne au problème de trouver des types de groupes protecteurs qui soient indépendants les uns des autres. Dans de nombreuses synthèses de peptides, la phase de récupération du peptide consiste à soumettre le peptide bloqué à un traitement avec un acide fort qui enlève tous les groupes protecteurs. Des acides tels que l'acide fluorhydrique, l'acide trifluorométhanesulfonique, trifluoroacétique sont par exemple employés. Cependant ce traitement n'est pas sans inconvénient. On a observé de nombreuses réactions secondaires, par exemple, lorsqu'on utilise les groupes protecteurs benzyle ou tertiobutyle, des carbocations benzyliques ou tertiobutyliques se forment et réagissent de façon tout à fait imprévisible avec les groupes fonctionnels qui ont été libérés, des réactions de déshydratation ou d'alkylation se produisent également. Il en résulte de grandes difficultés pour récupérer le peptide non modifié et ce d'autant plus qu'il est plus compliqué.

Il existait donc un besoin d'un procédé de préparation de peptides qui soit simple et qui ne présente pas les inconvénients des procédés antérieurs.

L'invention a pour objet un nouveau procédé de préparation de peptides en solution ou en phase solide qui est caractérisé

- en ce qu'on forme la liaison peptidique en condensant un premier dérivé d'un acide aminé ou d'un peptide à allonger dans lequel la fonction amine en $\alpha$ est protégée par un groupe choisi parmi le tert-butyloxycarbonyle (Boc), le 9-fluorénylméthyloxycarbonyle (Fmoc), le méthoxybenzyloxycarbonyle (Moz), l'$\alpha$,$\alpha$-diméthyl-3,5-diméthoxybenzyloxycarbonyle (Ddz) et le 2-nitrophénylsulfényle (Nps), et dans lequel le(s) groupe(s) fonctionnel(s) présent(s) et à protéger dans la ou les chaine(s) latérale(s) de l'acide aminé ou du peptide est(sont) bloqué(s) par le groupe Y de formule $R^1$-CH=CH-$CH_2$-, $R^1$ représentant un atome d'hydrogène ou un groupe alkyle en $C_1$ à $C_4$, sous forme de carbonates lorsque ce sont des groupes fonctionnels -OH alcool, de thiocarbonates ou de thiométhylènecarbamates lorsque ce sont des groupes fonctionnels -SH, d'éthers ou de carbonates lorsque ce sont des groupes fonctionnels -OH phénolique, de carbamates lorsque ce sont des groupes fonctionnels amino ou imino et d'esters lorsque ce sont des groupes fonctionnels -COOH, avec un second dérivé d'un acide aminé ou d'un peptide à allonger dans lequel la fonction acide -COOH en $\alpha$ est protégée sous la forme d'un groupe ester -$COOR^2$ dans lequel $R^2$ est un radical alkyle en $C_1$ à $C_{10}$, un radical aryle ou aralkyle substitué ou non sur le noyau aromatique ou le radical phénacyle, ou sous la forme d'un groupe amide -$CONR^3R^4$ dans lequel $R^3$ et $R^4$ identiques ou différents représentent un atome d'hydrogène, un radical alkyle en $C_1$ à $C_{10}$ ou un radical aryle ou aralkyle substitué ou non sur le noyau aromatique, les radicaux $R^2$, $R^3$ et/ou $R^4$ pouvant être reliés ou non à un polymère de type polystyrène ou polyacrylique, et dans lequel le(s) groupe(s) fonctionnel(s) présent(s) et à protéger dans la ou les chaine(s) latérale(s) de l'acide aminé ou du peptide est(sont) bloqué(s) par le groupe Y de formule $R^1$-CH=CH-$CH_2$-, $R^1$ représentant un atome d'hydrogène ou un groupe alkyle en $C_1$ à $C_4$, sous forme de carbonates pour les groupes fonctionnels -OH alcool, de thiocarbonates ou de thiométhylènecarbamates pour les groupes fonctionnels -SH, d'éthers ou de carbonates pour les groupes fonctionnels -OH phénolique, de carbamates pour les groupes fonctionnels amino ou imino et d'esters pour les groupes fonctionnels -COOH,
- on forme éventuellement une dernière liaison peptitique en condensant le peptide précédemment obtenu avec un dérivé d'acide aminé ou d'un peptide dans lequel la fonction amine en $\alpha$ est protégée si nécessaire par un groupe protecteur habituel et dans lequel le(s) groupe(s) fonctionnel(s) présent(s) et à protéger dans la ou les chaîne(s) latérale(s) de l'acide aminé ou du peptide est(sont) bloqué(s) par le groupe Y comme indiqué précédemment,
- on élimine si nécessaire le groupe protecteur de la fonction amine en $\alpha$ éventuellement présent et/ou les groupes protecteurs Y des groupes fonctionnels des chaînes latérales et/ou le groupe protecteur de la fonction acide en $\alpha$ ; les groupes protecteurs de la fonction amine en $\alpha$, autres que de type Y, et de la fonction acide en $\alpha$ étant

enlevés de façon connue, les groupes protecteurs Y étant enlevés tous en même temps par une catalyse douce au moyen d'un composé d'un métal noble tel qu'un métal du groupe du platine, ruthénium, rhodium, osmium, iridium, platine ou palladium.

Le procédé selon l'invention permet de préparer des peptides qui contiennent en partie ou totalement des restes d'acides aminés dont les chaînes latérales portent un ou plusieurs groupes fonctionnels réactifs.

Tous les acides aminés naturels ou synthétiques connus peuvent être utilisés et particulièrement ceux dont la chaîne latérale porte un ou plusieurs groupes fonctionnels ainsi que les peptides qui en sont constitués. Ils peuvent être sous forme D, L ou DL. Par exemple on peut citer comme acides aminés portant des groupes fonctionnels, l'arginine (Arg), l'acide aspartique (Asp), la cystéine (Cys), l'acide glutamique (Glu), l'histidine (His), la lysine (Lys) , l'hydroxylysine, l'hydroxyproline (Hyp), la sérine (Ser), la thréonine (Thr), le tryptophan (Trp), la tyrosine (Tyr), la thyroxine (Thy), l'ornithine (Orn), l'acide α-aminopimélique et l'acide α-aminosubérique.

Le procédé peut être réalisé en phase liquide ou en phase solide.

De préférence le groupe Y qui protège les groupes fonctionnels des chaînes latérales est le groupe de formule $CH_2$=CH-$CH_2$-, désigné par le terme All. Lorsque les groupes fonctionnels sont des groupes -OH phénolique, ils sont de préférence protégés sous forme d'éthers.

Les groupes protecteurs préférés de la fonction amine en α du premier dérivé d'acide aminé ou du peptide à allonger sont le groupe tert-butyloxycarbonyle (Boc) et le groupe 9-fluorénylméthyloxycarbonyle (Fmoc).

Ces dérivés α-amino protégés sont obtenus à partir des acides aminés en introduisant d'abord le groupe protecteur de la fonction amine en α et ensuite le ou les groupe(s) protecteur(s) Y ou inversement, selon des méthodes connues ou selon les procédés qui seront décrits plus loin.

La fonction acide en α de l'autre dérivé d'acide aminé ou du peptide à allonger est protégée sous forme d'esters ou d'amides par des groupes connus pour être stables lors de l'élimination des groupes protecteurs précédemment cités de la fonction amine en α et en particulier lors de l'élimination des groupes Boc et Fmoc protecteurs de cette fonction, comme indiqué dans les publications concernant les peptides telles qu'en particulier "Methoden der organischen Chemie" Houben-Weyl, vol 15/1 et /2, Syntheses von Peptiden, 1974; et "Principle of Peptide Synthesis" par M. Bodansky, Springer-Verlag, 1984. Le plus souvent la fonction acide est protégée sous forme d'esters par un radical alkyle en $C_1$ à $C_5$ ou benzyle.

Lorsqu'on utilise la synthèse en phase solide décrite par Merrifield, les groupes protecteurs de la fonction acide peuvent être reliés à une résine insoluble pendant les différentes opérations au moyen de groupes liants également stables dans les conditions de déprotection du groupe amino en α. De nombreuses combinaisons sont possibles et bien décrites dans la littérature comme par exemple dans "Solid-phase peptide synthesis : a silver anniversary report" par G. Barany & Co, 1987, Int. J. Peptide Protein Res. 30, 1987, 705-739 et dans "Solid phase peptide synthesis" par J.M. Stewart et J.D. Young (1984).

La résine est généralement un polystyrène, par exemple un polystyrène greffé par 1% de divinylbenzène ou de type polyacrylique par exemple un copolymère réticulé de N-acrylylpyrrolidine et de l'ester méthylique du N-acrylyl-β-alanine.

La préparation de ces dérivés dans lesquels la fonction acide en α et les groupes fonctionnels des chaînes latérales sont protégés, est réalisée selon les méthodes usuelles ou de préférence à partir des dérivés précédents α-aminoprotégés, par une réaction d'estérification ou d'amidification éventuellement suivie par une étape de fixation sur une résine si l'on souhaite opérer en phase solide, puis ensuite par une déprotection de la fonction amine en α.

La formation de la liaison peptidique entre les deux dérivés d'acides aminés ou de peptides s'effectue par les méthodes connues généralement au moyen de dérivés anhydrides, esters actifs ou de réactifs de couplage spécifiques. Ces méthodes sont tout à fait standardisées et elles ne seront pas décrites ici en détail. Elles sont en particulier développées dans les publications précédemment citées.

On peut éventuellement former une dernière liaison peptidique en fixant un dernier dérivé d'acide aminé ou d'un peptide dans lequel la fonction amine en α est protégée si nécessaire par un des groupes protecteurs connus habituellement utilisés en synthèse peptidique, par exemple tel qu'un des groupes protecteurs cités précédemment, le groupe benzyloxycarbonyle (Z) ou le groupe allyloxycarbonyle et dans lequel le(s) groupe(s) fonctionnel(s) présent(s) et à protéger dans la ou les chaîne(s) latérale(s) de l'acide aminé ou du peptide est (sont) bloqué(s) par le groupe Y comme indiqué précédemment. Par exemple, des composés tels que l'acide pyroglutamique ou des dérivés N-acétyl d'acides aminés ou peptides peuvent être utilisés comme composé N-terminal.

Pendant la formation des liaisons peptidiques les groupes fonctionnels ne participant pas à la réaction restent bloqués.

A la fin de la synthèse, les groupes protecteurs de la fonction amine en α et/ou de la fonction acide en α et/ou des groupes fonctionnels des chaînes latérales du peptide formé peuvent être éliminés si nécessaire, de façon indépendante et dans un ordre quelconque. Le groupe protecteur de la fonction amine en α, est généralement enlevé par acidolyse modérée pour les groupes Boc, Moz, Ddz, Nps ou par clivage basique pour le groupe Fmoc.

De préférence lorsque le groupe protecteur est le groupe Boc, il est éliminé au moyen d'un acide fort, tel que l'acide trifluoroacétique dans du dichlorométhane ou l'acide chlorhydrique gazeux dans du dioxanne. Le groupe Fmoc est lui généralement éliminé par une amine secondaire dans un solvant et de préférence par de la pipéridine dans le diméthylformamide ou dans la N-méthylpyrrolidone.

Les groupes de type allylique, tels que ceux qui protègent les groupes fonctionnels des chaînes latérales, sont enlevés tous en même temps, en particulier au moyen d'une quantité catalytique d'un composé du rhodium (I) et de préférence d'un composé du palladium (O) ou (II), dans un solvant ou un système de solvants approprié en présence d'accepteurs nucléophiles ou de donneurs de protons comme par exemple la morpholine, la dimédone ou d'autres composés à hydrogène mobile aisément déprotonables.

Un procédé convenant bien pour cette déprotection consiste à faire réagir le peptide avec l'hydrure de tributylétain en présence d'une quantité catalytique de palladium (O) tétrakis(triphénylphosphine) ou de palladium (II) dichlorobis (triphénylphosphine) et d'un composé faiblement acide tel que l'acide acétique, le p-nitrophénol ou l'acétate de pyridinium ou d'eau, dans un milieu solvant. Des solvants très variés peuvent être utilisés tels que benzène, toluène, éther diéthylique, tétrahydrofuranne, dichlorométhane, acétone, acétate d'éthyle et diméthylformamide.

On peut également effectuer cette déprotection au moyen d'une quantité catalytique de palladium (O) tétrakis (triphénylphosphine) en présence de N,N-diméthylsilylamine et d'acétate de triméthylsilyle dans un milieu solvant à bas point d'ébullition et aprotique tel que le dichlorométhane, en faisant réagir ensuite le composé obtenu avec du méthanol.

L'élimination du groupe protecteur de la fonction acide en $\alpha$ du peptide est généralement effectuée de façon classique, par exemple par acidolyse avec de l'acide fluorhydrique, de l'acide trifluorométhanesulfonique ou de l'acide trifluoroacétique.

Lorsqu'on veut récupérer le peptide libre de toute protection, on préfère enlever en premier le groupe protecteur de la fonction amine en $\alpha$, s'il n'est pas de type allylique. Pendant cette opération les groupes protecteurs de type allylique dans les chaînes latérales ne sont pas attaqués. On enlève ensuite en même temps tous les groupes protecteurs de type allylique et en dernier s'il n'a pas été éliminé au cours d'un des traitements précédents on enlève le groupe protecteur de la fonction acide en $\alpha$.

Le procédé selon l'invention permet d'obtenir des peptides très purs. L'élimination du groupe protecteur de la fonction amine en $\alpha$ peut s'effectuer sans modifier les groupes protecteurs des groupes fonctionnels des chaînes latérales et par conséquent sans apparition de réactions secondaires et les groupes fonctionnels des chaînes latérales sont tous libérés par un même traitement.

L'invention a également pour objet les nouveaux dérivés d'acides aminés mis en oeuvre dans le procédé.

Ces nouveaux dérivés ont la formule générale

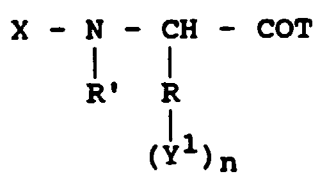

(I)

dans laquelle

X représente le groupe tert-butyloxycarbonyle (Boc), 9-fluorénylméthyloxycarbonyle (Fmoc), méthoxybenzyloxycarbonyle (Moz), $\alpha,\alpha$-diméthyl-3,5-diméthoxybenzyloxycarbonyle (Ddz) ou 2-nitrophénylsulfényle (Nps),

T représente le groupe OH, le radical $-OR^2$ dans lequel $R^2$ représente un radical alkyle en $C_1$ à $C_{10}$, un radical aryle ou aralkyle substitué ou non sur le noyau aromatique ou le radical phénacyle ou T représente le radical $-NR^3R^4$ dans lequel $R^3$ et $R^4$ identiques ou différents représentent un atome d'hydrogène, un radical alkyle en $C_1$ à $C_{10}$ ou un radical aryle ou aralkyle substitué ou non sur le noyau aromatique,

R' représente un atome d'hydrogène et R représente le reste de la chaîne latérale d'un acide aminé qui contient au moins un groupe fonctionnel -OH,-SH, >NH, =NH, $-NH_2$, -COOH ou bien R' et R sont liés et forment le reste d'un acide aminé cyclique qui porte au moins l'un des groupes fonctionnels précédents,

$Y^1$ représente le radical

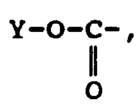

dans lequel Y a pour formule $R^1$-CH=CH-$CH_2$-, $R^1$ représentant un atome d'hydrogène ou un groupe alkyle en $C_1$ à $C_4$, lorsque $Y^1$ remplace l'atome d'hydrogène du ou des groupe(s) fonctionnel(s) -OH, -SH, >NH, =NH, ou -$NH_2$ contenu(s) dans le reste R ou le reste formé par R lié à R', sauf lorsque X est le radical tert-butyloxycarbonyle, T représente le groupe -OH et R représente le reste de la chaîne latérale de la lysine,
ou $Y^1$ représente le groupe

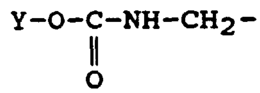

(appelé également dans la présente description méthylènecarbamate), Y ayant la signification indiquée ci-dessus, lorsque $Y^1$ remplace l'atome d'hydrogène du ou des groupe(s) fonctionnel(s) -SH contenu(s) dans le reste R ou le reste formé par R lié à R',
ou bien $Y^1$ représente le radical Y ayant la signification indiquée ci-dessus, lorsque $Y^1$ remplace l'atome d'hydrogène du ou des groupe(s) fonctionnel(s) -COOH et/ou -OH phénolique contenu(s)dans le reste R ou le reste formé par R lié à R', sauf lorsque X est le radical tert-butyloxycarbonyle, T représente le groupe -OH et R représente le reste de la chaîne latérale de l'acide aspartique, de l'acide glutamique ou de la tyrosine,
n = 1 lorsque R ne contient qu'un seul groupe fonctionnel ou n = 1 ou 2 lorsque R contient deux groupes fonctionnels.

Les nouveaux dérivés selon l'invention peuvent être sous forme D, L ou DL. Lorsque T représente le groupe OH, ils peuvent être sous forme de sels avec les composés habituellement utilisés en synthèse peptidique, par exemple avec la dicyclohexylamine.

Les radicaux R seuls ou en combinaison avec R' représentent les restes de tous les acides aminés connus naturels ou synthétiques qui contiennent au moins un groupe fonctionnel -OH, -SH, >NH, =NH, -NH2,- COOH dans leur chaîne latérale. Parmi les acides aminés qui contiennent de tels groupes on peut en particulier citer : l'arginine (Arg), l'acide aspartique (Asp), la cystéine (Cys) , l'acide glutamique (Glu), l'histidine (His), la lysine (Lys), l'hydroxylysine, l'hydroxyproline (Hyp), la sérine (Ser), la thréonine (Thr), le tryptophan (Trp),la tyrosine (Tyr), la thyroxine (Thy), l'ornithine (Orn), l'acide $\alpha$-aminopimélique et l'acide $\alpha$-aminosubérique.

Les dérivés préférés sont ceux dans lesquels Y représente le radical allyle $CH_2$=CH-$CH_2$-. Plus particulièrement ce sont ceux dans lesquels X représente le groupe Boc ou Fmoc et T est le groupe -OH ou le radical -$OR^2$ dans lequel $R^2$ représente un radical alkyle en $C_1$ à $C_5$ ou le radical benzyle.

Comme composés particulièrement utiles on peut citer les composés suivants : Boc-Ser(Alloc)-OH, Boc-Thr(Alloc)-OH, Boc-Thr(Alloc)-$OCH_3$, Boc-His (Alloc)-OH, Boc-Arg(Alloc)-OH, Boc-Arg$(Alloc)_2$-OH, Boc-Cys(Alloc)-OH, Fmoc-Asp(OAll)-OH, Fmoc-Glu(OAll)-OH, Fmoc-Cys-(Alloc)-OH, Fmoc-Lys-(Alloc)-OH, Fmoc-Tyr(All)-OH, Fmoc-Ser (Alloc)-OH, Fmoc-Thr(Alloc)-OH, Fmoc-His(Alloc)-OH, Fmoc-Arg-(Alloc)-OH, Fmoc-Arg$(Alloc)_2$-OH, Boc-Cys(Allocam)-OH Fmoc-Cys(Allocam)-OH. La désignation Alloc représente le groupe

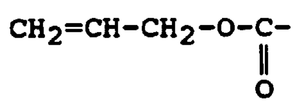

et la désignation Allocam représente le groupe

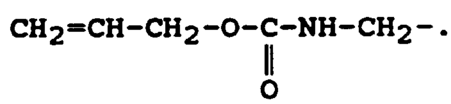

Ces nouveaux dérivés peuvent être préparés de différentes façons.

Lorsque $Y^1$ représente le radical

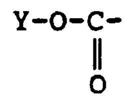

dans lequel Y a pour formule $R^1$-CH=CH=$CH_2$-, $R^1$ ayant les significations précédemment indiquées, ils sont de préférence préparés par réaction du chloroformiate de formule

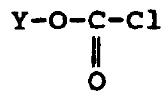

sur le composé de formule

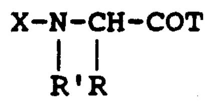

dans lesquelles Y, X, T, R et R' ont les significations précédemment indiquées, en milieu anhydre ou en milieu aqueux neutre ou basique selon le(s) groupe(s) fonctionnel(s) présent(s) dans le reste R ou dans le reste formé par R lié à R', à une température comprise entre 0° et 50°C.

En particulier lorsque le groupe fonctionnel latéral à protéger est un groupe amino ou imino, les deux composés de départ sont mis à réagir dans un milieu aqueux à pH basique compris entre 7 et 12.

Lorsque le radical R est le reste de la sérine les composés sont mis à réagir en milieu aqueux neutre.

Lorsque R est le reste de la thréonine, de préférence le milieu est anhydre et on fait réagir le chloroformiate

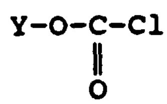

sur le dérivé de la thréonine de formule

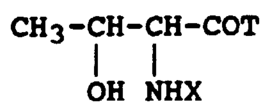

dans laquelle X a les significations indiquées précédemment et T représente le radical -$OR^2$ ou -$NR^3R^4$, $R^2$, $R^3$ et $R^4$ ayant les significations indiquées précédemment, en présence de diméthylaminopyridine puis on fait éventuellement un traitement enzymatique pour libérer la fonction acide en $\alpha$.

Lorsque $Y^1$ représente un groupe Y tel que décrit précédemment et qu'il remplace l'atome d'hydrogène d'un groupe -COOH ou d'un groupe -OH phénolique, on fait réagir un composé de formule Y-Br en milieu basique sur le complexe cuivrique de l'acide aminé, éventuellement dans un solvant miscible à l'eau, de préférence l'alcool méthylique ou éthylique, à une température comprise entre 0° et 50°C puis on introduit après élimination du cuivre, le groupe protecteur X selon les procédés connus, par exemple au moyen du fluoroformiate, du chloroformiate ou du tétrachloroéthylcarbonate de X et éventuellement on transforme la fonction -COOH en $\alpha$, en ester -$COOR^2$ ou en amide -$CONR^3R^4$, $R^2$, $R^3$ et $R^4$ ayant les significations précédemment indiquées.

Lorsque $Y^1$ représente le groupe

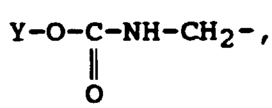

Y ayant la signification précédemment indiquée et que $Y^1$ remplace l'atome d'hydrogène d'un groupe -SH, comme par exemple dans la cystéine, on fait réagir l'hydroxyméthylcarbamate du groupe de type allyle sur l'acide aminé ou de préférence son chlorhydrate, en milieu acide à une température comprise entre 0° et 50°, éventuellement dans un solvant aprotique tel que le dichlorométhane puis on introduit le groupe protecteur X selon les procédés connus, par exemple comme précédemment indiqué et ensuite éventuellement on transforme la fonction -COOH en α comme précédemment.

Les exemples qui suivent illustrent l'invention sans toutefois la limiter.

Dans tous les exemples les acides aminés et leurs dérivés sont sous forme (L).

Exemple 1 : Préparation de Boc-His(Alloc)-OH

5,1 g de Boc-His-OH (0,02 mol) sont dissouts dans 10 ml de NaOH 2N et 10 ml d'eau. Le mélange est refroidi à 0°C et agité. On ajoute 2 ml de chloroformiate d'allyle (0,019 mol) petit à petit en maintenant le pH à 7-7,5 avec NaOH 2N. Puis on continue à agiter à 0°C pendant 1 heure. La solution est lavée une fois par de l'éther éthylique ($Et_2O$) puis acidifiée avec HCl concentré jusqu'à pH 2-3. Le produit est extrait trois fois par EtOAc, lavé par $H_2O$, séché sur $MgSO_4$. On obtient après évaporation du solvant, 3,9 g du produit souhaité sous forme d'un solide-mousse (rendement 57%).

Deux taches sont obtenues par chromatographie en couche mince (CCM) dans le solvant $CHCl_3/CH_3OH/AcOH$ (90/8/2) par révélation au réactif ninhydrine (après le clivage habituel du groupe Boc par vaporisation d'acide chlorhydrique). Les structures des deux isomères π et τ sont confirmées par RMN.

Exemple 2 : Préparation de Boc-Arg$(Alloc)_2$-OH et Boc-Arg(Alloc)-OH

5,48 g de Boc-Arg-OH (0,02 mol) sont dissouts dans un mélange de 40 ml de NaOH 2N et 12 ml de dioxanne à 0°C et agité. Le pH de la solution est environ 14. On ajoute jusqu'à pH 12 environ 5 ml de chloroformiate d'allyle. Puis on continue à ajouter 5 ml de chloroformiate d'allyle en ajustant le pH autour de 11,5-12 par addition de NaOH 2N. On maintient l'agitation 2 h après l'addition du chloroformiate.

La phase aqueuse est lavée deux fois par $Et_2O$ et acidifiée ensuite par HCl concentré jusqu'à pH 3. On extrait le produit trois fois avec EtOAc. La phase organique contient 3 produits, le produit de départ, le produit avec un seul groupe Alloc et le produit avec deux groupes Alloc. Elle est lavée par $H_2O$ à pH 3 pour éliminer le produit de départ puis lavée par $H_2O$ à pH 6 pour obtenir le dérivé monoalloc dans la phase aqueuse.

La phase organique est ensuite séchée sur $MgSO_4$. Après évaporation du solvant, on obtient 0,8 g de Boc-Arg $(Alloc)_2$-OH sous forme d'huile.

$[\alpha]_D^{20}$ : + 2,1(c 1,$CH_3OH$)

La structure est confirmée par RMN.

La phase aqueuse précédente est saturée par NaCl. On extrait trois fois le produit Boc-Arg(Alloc)-OH avec EtOAc. La phase organique est séchée sur $MgSO_4$. On obtient 0,3 g de Boc-Arg(Alloc)-OH sous forme de mousse.
(structure confirmée par RMN)

$[\alpha]_D^{20}$ : - 3,8 (c 1,$CH_3OH$)

Chaque produit donne une seule tache en CCM dans le solvant $CHCl_3/CH_3OH//AcOH$ (90/8/2) par révélation au réactif ninhydrine.

Exemple 3 : Préparation de Boc-Ser(Alloc)-OH

4,1 g de Boc-Ser-OH (0,02 mol) sont dissouts dans 15 ml de NaOH 2N. On ajoute alternativement 9 ml de chloroformiate d'allyle (0,085 mol) et 80 ml de NaOH 2N dix fois de suite. On laisse réagir une heure.

Le mélange réactionnel est acidifié par HCl concentré jusqu'à pH 2-3. On extrait la phase organique avec EtOAc (3 fois) puis on lave la phase organique par de l'eau jusqu'à élimination complète de la Boc-Ser-OH.

Le solvant est évaporé et on obtient une huile (0,8 g). Cette huile est dissoute dans l'éther éthylique. On ajoute 1 équivalent de dicyclohexylamine (DCHA) puis de l'hexane. On obtient 1 g du produit souhaité sous forme de sel de DCHA (structure confirmée par RMN).

Point de fusion : 111-113°C.

$[\alpha]_D^{20}$ : + 19,1 (c 1,$CH_3OH$)

Une tache en CCM dans le solvant $CHCl_3$/MeoH/AcOH (90/8/2) après révélation au réactif ninhydrine.

Exemple 4 : Préparation de Boc-Thr(Alloc)-OH

8,24 g de Thr-OMe.HCl (0,05 mol) sont dissouts dans 20 ml de NaOH 2N. On ajoute 10 ml d'eau et 30 ml d'acétone. On agite. On ajoute goutte à goutte 20 g de Boc-F (40% dans du diméthoxyéthane (DME), 0,065 mol) en maintenant

le pH à 7-7,5 par addition de NaOH 2N et la température de la réaction à 10-15°C. On continue l'agitation pendant 15 min après l'addition de Boc-F.

Le produit est extrait trois fois par de l'éther de tert-butyle et de méthyle (TBME) et la phase organique lavée par de l'eau puis séchée sur $MgSO_4$. On obtient après évaporation du solvant une huile incolore. On lave l'huile avec de l'hexane. On obtient 7 g (0,03 mol) de Boc-Thr-OMe. On les dissout dans 50 ml de $CH_2Cl_2$ sec à 0°C. On ajoute 3,66 g de diméthylaminopyridine (DMAP) (0,03 mol) et 3,82 ml de chloroformiate d'allyle (0,036 mol). Le mélange est agité à la température ambiante pendant 24 h. Le solvant est évaporé. On ajoute de l'éther éthylique et de l'eau. La phase organique est lavée par deux fois HCl 1N, deux fois $NaHCO_3$ à 5% et deux fois NaCl saturé. Le produit pur est séparé par chromatographie sur silice (solution éluante, $CHCl_3$). On obtient 4,6 g de Boc-Thr(Alloc)-OMe pur (rendement 48%).

1,123 g de Boc-Thr(Alloc)-OMe (3,5 mol) sont dissouts dans 10 ml d'EtOH et 20 ml de $H_2O$. Puis on ajoute 50 mg de cystéine.HCl.$H_2O$. Le pH est porté à 6,2 par NaOH 0,1 N. Le mélange réactionnel est thermostaté à 35°C ± 2°C. On ajoute 50 mg de papaïne brute. Le pH du mélange est maintenu autour de 6,2 par l'addition de NaOH 0,1 N. 35 ml (3,5 mol) de NaOH 0,1 N ont été consommés. Le pH est porté à 8,5. La solution aqueuse est lavée deux fois par TBME puis acidifiée par HCl concentré jusqu'à pH 2-3. On extrait le produit au TBME. La phase organique est lavée une fois par $H_2O$ et filtré sur célite puis séchée sur $MgSO_4$. Le solvant est évaporé. On récupère 0,7 g de produit sous forme d'huile.

L'huile est dissoute dans l'éther. On ajoute 1 équivalent de DCHA puis de l'hexane. On laisse le produit au froid pendant quelques heures. On récupère 0,53 g du produit attendu sous forme de sel de DCHA qui est un solide blanc (rendement 31%). Point de fusion : 108-109°C.

$[\alpha]_D^{20}$ : + 22 (c 1,$CH_3OH$)

Une tache par CCM dans le solvant $CHCl_3$/MeOH/AcOH (90/8/2) par révélation au réactif ninhydrine.

Exemple 5 : Préparation de Boc-Cys(Alloc)-OH

2,21 g de Boc-Cys-OH (10 mmol) sont traités par NaOH 2N et de l'eau. Le pH est maintenu à 8. 1,6 ml de chloroformiate d'allyle (15 mmol) et du NaOH 2N sont ajoutés en même temps en maintenant le pH à 7-8. A la fin de l'addition, le mélange est acidifié jusqu'à pH 2,5 puis extrait trois fois avec AcOEt. La phase AcOEt est séchée sur $MgSO_4$. On obtient une huile après évaporation du solvant. L'huile est coévaporée plusieurs fois avec de l'éther et de l'hexane. L'huile visqueuse est conservée à température ambiante. Le produit se solidifie. On récupère 2,8 g du produit attendu sous forme d'un solide blanc, par filtration (rendement 92%). La structure est confirmée par RMN. Point de fusion : 69-71°C.

$[\alpha]_D^{20}$ : - 47,3 (c 1,$CH_3OH$)

Exemple 6 : Préparation de Fmoc-Lys(Alloc)-OH

1,15 g de Lys(Alloc)-OH (5 mmol) est dissout dans 10 ml d'acétone, 10 ml de NaOH 0,5 N et 10 ml d'eau. Le pH est environ 9. On ajoute 2,23 g de carbonate de 9-fluorénylméthyle et de tétrachloroéthyle (Fmoc-OTCE) en ajustant le pH à 7-8 par l'addition de NaOH 2N. A la fin de la réaction, le pH est porté à 9-9,5. La phase aqueuse est lavée trois fois par de l'éther puis acidifié jusqu'à pH ≃ 2,5. On extrait le produit trois fois avec EtOAc. La phase EtOAc est séchée sur $MgSO_4$. Après évaporation du solvant on obtient une mousse. La mousse est dissoute dans l'éther. On ajoute de l'hexane puis on passe le mélange à l'ultrason. Le précipité est récupéré par filtration. On obtient 1,82 g du produit souhaité (rendement 80%). Point de fusion : 88-90°C.

$[\alpha]_D^{20}$ : - 11,3 (c 1,diméthylformamide (DMF))

Exemple 7 : Préparation de Fmoc-Tyr(All)-OH

0,44 g de Tyr(All)-OH (2 mmol) sont mis en suspension dans un mélange de $H_2O$/acétone(1/1). Le pH est ajusté à 8 par addition de NaOH 0,5 N. 0,9 g de Fmoc-OTCE sont ajoutés en une fois. Le pH est maintenu à 7-8. Le mélange réactionnel passe de trouble à limpide puis devient une émulsion. On continue l'agitation pendant 12 h à température ambiante. Le mélange réactionnel est acidifié jusqu'à pH ≃ 2,5 et extrait par trois fois par AcOEt. On ajoute de l'eau. Le pH est ajusté à 9,5. La phase aqueuse est séparée et acidifié jusqu'à pH ≃ 2,5 et extraite trois fois par EtOAc. Le produit est séché sur $MgSO_4$ puis est précipité dans EtOAc/$Et_2O$/hexane. On obtient 100 mg du produit souhaité.
Point de fusion : 75-78°C.

$[\alpha]_D^{20}$ : - 21,3 (c 1,DMF)

Exemple 8 : Préparation de Fmoc-Glu(OAll)-OH

1,11 g de Glu(OAll)-OH.HCl (5 mmol) est dissout dans 10 ml de NaOH 0,5 N, 20 ml d'eau et 20 ml d'acétone. 2,64

g de Fmoc-OTCE sont ajoutés en une fois. Le pH est maintenu à 7,5 par addition de NaOH 1 N. A la fin de la réaction le pH est ajusté à 8,5. La phase aqueuse est lavée quatre fois par $Et_2O$ puis acidifiée jusqu'à pH ≃ 2,5. Le produit est extrait trois fois par $Et_2OAc$. La phase organique obtenue est lavée par trois fois $H_2O$ (pH 2,5) et $H_2O$ (pH 6) puis séchée sur $MgSO_4$. On évapore le solvant. Le produit est dissout dans $Et_2O$. De l'hexane est ajouté puis le mélange trouble est passé à l'ultrason. Un solide précipite. On obtient 1 g du produit souhaité (rendement 48%).
Point de fusion : 102-105°C.
$[\alpha]_D^{20}$ : - 17,1 (c 1,DMF)

Exemple 9 : Préparation de Fmoc-Asp(OAll)-OH

2,1 g de Asp(OAll)-OH.HCl (10 mmol) sont mis en suspension dans 30 ml de diméthylsulfoxyde (DMSO). On ajoute 5,02 ml de $Et_3N$ (36 mmol). Le mélange devient visqueux. 8,18 g de Fmoc-OTCE sont ajoutés en une fois. Le mélange réactionnel est agité pendant 18 h à 40°C sous argon. On ajoute de l'eau et EtOAc. Le pH est ajusté à 9. La phase aqueuse est séparée et ensuite acidifiée jusqu'au pH ≃ 2,5. on extrait le produit par trois fois EtOAc. La phase EtOAC est séchée sur $MgSO_4$. Une mousse est obtenue après évaporation du solvant. Le produit est précipité dans MeOH/$Et_2O$/hexane. On obtient 0,53 g du produit souhaité.
Point de fusion : 140°C.
$[\alpha]_D^{20}$ : + 5,9 (c 1,DMF)

Exemple 10 : Préparation de Fmoc-Arg$(Alloc)_2$-OH

0,44 g de Boc-Arg$(Alloc)_2$-OH (1 mmol) est traité par 10 ml d'acide trifluoroacétique et de $CH_2Cl_2$ (1/1) pendant 30 min. Puis le solvant est évaporé à l'évaporateur rotatif. Le résidu est coévaporé avec deux fois $CH_2Cl_2$. Le résidu est dissout dans 10 ml d'acétone et 30 ml d'eau. Le pH est ajusté à 9 par NaOH 2N. On ajoute 1,2 éq. de Fmoc-OTCE en maintenant le pH à 8-9. A la fin de l'addition on obtient une émulsion. La phase aqueuse trouble est lavée par de l'éther quatre fois puis acidifiée par HCl concentré jusqu'à pH ≃ 2,5. Le produit est extrait par trois fois AcOEt. Cette phase organique est séchée sur $MgSO_4$. On évapore le solvant et on obtient une huile. Cette huile est traitée par $Et_2O$-hexane, passée à l'ultrason. On sépare 100 mg du produit souhaité sous forme d'un solide blanc.
Point de fusion : 78-80°C.
$[\alpha]_D^{20}$ : - 9,1 (c 1,DMF)

Exemple 11 : Préparation de Boc-Cys(Allocam)-OH

a) Préparation de HCl.Cys(Allocam)-OH

On dissout 10 g de chlorhydrate de cystéine (0,063 mol) dans 60 ml d'acide trifluoroacétique. On ajoute lentement une solution d'hydroxyméthylcarbamate d'allyle (9,04 g, 0,069 mol) dans 60 ml de $CH_2Cl_2$. On agite le mélange réactionnel à température ambiante pendant 10 min. Puis on évapore le solvant. On obtient un solide blanc. On ajoute 1,1 équivalent de HCl (1 N). On évapore la solution puis le résidu est coévaporé avec $C_2H_5OH$. On obtient le produit attendu sous forme d'un solide qui est séché sous vide. Le rendement est de 95%.

b) Préparation de Boc-Cys(Allocam)-OH

On dissout 10 mmol de HCl.Cys (Allocam)-OH préparée comme précédemment dans 20 ml d'eau à pH 9. On ajoute lentement environ 12 mmol de Boc-F (40% en volume dans du DME) en maintenant le pH à 9 par addition de NaOH (2N). Après cette addition on continue à agiter pendant 30 min. On lave la phase aqueuse 2 fois avec de l'éther éthylique puis on acidifie jusqu'à pH 3. On extrait le produit avec AcOEt, on lave avec $H_2O$ et on sèche sur $MgSO_4$. On évapore le solvant et on obtient le produit attendu sous forme d'une huile.

Exemple 12 : Préparation de Fmoc-Cys(Allocam)-OH

On dissout 10 mmol de HCl.Cys(Allocam)-OH dans 50 ml d'eau et 5 ml de dioxane. On ramène le pH à 11,5 par addition de NaOH (2N). On ajoute goutte à goutte 0,9 mmol de Fmoc-F (30% en volume dans du DME) en maintenant le pH à 11,5 par addition de NaOH (2N). On continue à agiter le mélange réactionnel pendant 1 h après cette addition. On extrait le mélange 2 fois avec de l'éther éthylique et on acidifie jusqu'à pH 3 avec HCl concentré. On extrait le produit 3 fois avec AcOEt, on lave avec $H_2O$ puis on sèche sur $MgSO_4$. On obtient le produit attendu avec un rendement de 75%.

Exemple 13 : Préparation de Arg-Ser-Tyr-Asp-Gly-Leu

La préparation est effectuée en phase solide avec les cycles suivants :
1er cycle : 0,5 g (0,71 méq/g) de résine Boc-Leu-PAM-P (PAM = phénylacétamidométhyl, P = polystyrène - 1% divinylbenzène) est placé dans un réacteur agité.

On lave deux fois avec $CH_2Cl_2$ et on filtre à chaque fois. On traite avec 50% en volume d'acide trifluoroacétique (TFA) dans $CH_2Cl_2$ (1 min puis 30 min) et on filtre. On lave avec de l'isopropanol puis on filtre. On lave deux fois avec $CH_2Cl_2$ et on filtre. On ajoute 2 éq. (314 mg) de BOP (benzotriazolyl-N-oxytrisdiméthylaminophosphoniumhexafluorophosphate), 2 éq. (124 mg) de Boc-Gly, 7,5 ml de $CH_2Cl_2$ et 0,4 ml (6,6 éq) de diéthylisopropylamine (DIEA). Le pH est ajusté à 9 avec une petite quantité supplémentaire de DIEA si nécessaire.

Le test de Kaiser est négatif après 30 min de couplage.

On filtre et on lave 2 fois avec $CH_3OH$ et 2 fois avec $CH_2Cl_2$ en filtrant à chaque fois. La résine est prête pour le second cycle. Les mêmes opérations sont répétées pour chacun des cycles en changeant seulement la nature de l'acide aminé.

2ème cycle : 2 éq. (194 mg) de Boc-Asp(OAll)-OH
3ème cycle : 2 éq. (228 mg) de Boc-Tyr(All)-OH
4ème cycle : 2 éq. (334 mg) de Boc-Ser(Alloc)-OH.DCHA
5ème cycle : 2 éq. (332 mg) de Boc-Arg$(Alloc)_2$-OH

On traite ensuite avec 50% de TFA dans $CH_2Cl_2$ 1 min puis 30 min. On lave avec de l'isopropanol (1 fois), $CH_2Cl_2$ (2 fois), 10% en volume d'acide acétique (AcOH) dans $CH_2Cl_2$, $CH_2Cl_2$ (2 fois) en filtrant à chaque fois. On traite ensuite avec 49 mg de $PdCl_2(PPh_3)_2$ puis 2,5 éq. de AcOH et 7 ml de $CH_2Cl_2$ puis on ajoute 2 éq. par rapport aux groupes allyle de $Bu_3SnH$. On secoue le mélange pendant 10 min. On lave avec $CH_2Cl_2$ et on filtre (2 fois). On ajoute 25 mg de $PdCl_2$ $(PPh_3)_2$, 1,5 éq. de AcOH et 7,5 ml de $CH_2Cl_2$ puis on ajoute 1 éq. de $Bu_3SnH$ et on secoue le mélange pendant 10 min. On lave ensuite avec $CH_2Cl_2$ (5 fois), $CH_3OH$ (5 fois), $CH_2Cl_2$ (5 fois) en filtrant à chaque fois et on sèche la résine sous vide pendant 2 heures. On obtient ainsi 0,65 g de Peptide-PAM-P. On sépare le peptide de la résine par 10% d'acide fluorhydrique dans l'anisole. Après évaporation de HF on extrait le peptide de la résine au moyen d'une solution aqueuse d'acide acétique à 10%. La solution contenant le peptide est lyophilisée. Le peptide est obtenu avec un rendement de 52% et une pureté déterminée par chromatographie en phase liquide haute pression (HPLC) supérieure à 80%.

Exemple 14 : Préparation de Fmoc-Ala-Arg-Gly

La préparation est effectuée en phase solide. La résine Boc-Gly-P' (P' = résine "Merrifield", polystyrène chlorométhylé réticulé par 1% de divinylbenzène) est traitée par une solution de TFA (50% en volume) dans $CH_2Cl_2$ pendant 30 min. Après lavage avec de l'isopropanol (iPrOH), du $CH_2Cl_2$ puis du diméthylformamide (DMF), on ajoute 2 équivalents de Boc-Arg$(Alloc)_2$-OH, 2 équivalents de l'agent de couplage O-benzotriazolyl-N-tétraméthyluronium tétrafluoroborate (TBTU). On ajoute ensuite de la diisopropyléthylamine ($iPr_2NEt$) jusqu'à pH 9. On agite le mélange pendant 30 min. Le test de Kaiser est négatif. On lave deux fois la résine avec $CH_3OH$ puis $CH_2Cl_2$. On enlève le groupe Boc de Boc-Arg $(Alloc)_2$Gly-P' par traitement avec une solution de TFA (à 50% en volume) dans $CH_2Cl_2$. On couple ensuite de la même façon Fmoc-Ala-OH sur la résine.

On mélange la résine obtenue avec du $PdCl_2(PPh_3)_2$ (4% en mole par fonction allyle) et avec AcOH (2,5 équivalents) dans $CH_2Cl_2$. On ajoute ensuite $Bu_3SnH$ (2 équivalents) en une seule fois. On agite le mélange pendant 10 min. On filtre la résine, on la lave deux fois avec $CH_2Cl_2$, $CH_3OH$ puis $CH_2Cl_2$. On répète l'opération deux fois encore. On traite ensuite la résine avec une solution de TFA saturé par HBr pendant 2 h à la température ambiante. Après filtration et évaporation du solvant, on obtient le tripeptide avec un rendement de 78%.

Exemple 15 : Préparation de Z-Ala-Lys-Gly-$NH_2$

La préparation est effectuée en phase solide. On traite la résine Boc-Gly-P' par une solution de TFA (50% en volume) dans $CH_2Cl_2$ pendant 30 min. Après lavage une fois avec de l'iPrOH, deux fois avec $CH_2Cl_2$ puis avec du DFM, on ajoute 2 équivalents de Fmoc-Lys(Alloc)-OH dans du DMF, et 2 équivalents de TBTU dans du DMF, on ajuste le pH du milieu réactionnel à 9 par addition de $iPr_2NEt$ (environ 6 équivalents). On suit la réaction de couplage avec le test de Kaiser. Elle est terminée au bout de 30 min. On lave la résine deux fois avec $CH_3OH$ puis deux fois avec $CH_2Cl_2$. On enlève le groupe Fmoc de Fmoc-Lys (Alloc)-Gly-P' avec un mélange de pipéridine (à 30% en volume) et de DMF pendant 10 min. Puis on couple de la même façon Z-Ala-OH sur la résine.

On met la résine en suspension dans un mélange de $CH_2Cl_2$, $PdCl_2(PPh_3)_2$ (4% en mole par fonction allyle) et

on ajoute AcOH (2,5 équivalents). Puis en une seule fois on ajoute $Bu_3SnH$ (2 équivalents). On agite le mélange pendant 10 min. On lave la résine deux fois avec $CH_2Cl_2$, deux fois avec $CH_3OH$ puis deux fois avec $CH_2Cl_2$. On répète une fois l'opération de déprotection. On traite la résine Z-Ala-Lys-Gly-P' par $CH_3OH$ saturé par $NH_3$ pendant 3 jours. On obtient le tripeptide après filtration et évaporation du solvant avec un rendement de 65%.

## Revendications

1. Procédé de préparation de peptides en solution ou en phase solide caractérisé :

   - en ce qu'on forme la liaison peptidique en condensant un premier dérivé d'un acide aminé ou d'un peptide à allonger dans lequel la fonction amine en α est protégée par un groupe choisi parmi le tert-butyloxycarbonyle (Boc), le 9-fluorénylméthyloxycarbonyle (Fmoc), le méthoxybenzyloxycarbonyle (Moz), l'α,α-diméthyl-3,5-diméthoxybenzyloxycarbonyle (Ddz) et le 2-nitrophénylsulfényle (Nps) , et dans lequel le(s) groupe(s) fonctionnel(s) présent(s) et à protéger dans la ou les chaîne(s) latérale(s) de l'acide aminé ou du peptide est(sont) bloqué(s) par le groupe Y de formule $R^1$-CH=CH-$CH_2$-, $R^1$ représentant un atome d'hydrogène ou un groupe alkyle en $C_1$ à $C_4$, sous forme de carbonates lorsque ce sont des groupes fonctionnels -OH alcool, de thiocarbonates ou de thiométhylènecarbamates lorsque ce sont des groupes fonctionnels -SH, d'éthers ou de carbonates lorsque ce sont des groupes fonctionnels -OH phénolique, de carbamates lorsque ce sont des groupes fonctionnels amino ou imino et d'esters lorsque ce sont des groupes fonctionnels -COOH, avec un second dérivé d'un acide aminé ou d'un peptide à allonger dans lequel la fonction acide -COOH en α est protégée sous la forme d'un groupe ester -$COOR^2$ dans lequel $R^2$ est un radical alkyle en $C_1$ à $C_{10}$, un radical aryle ou aralkyle substitué ou non sur le noyau aromatique ou le radical phénacyle, ou sous la forme d'un groupe amide -$CONR^3R^4$ dans lequel $R^3$ et $R^4$ identiques ou différents représentent un atome d'hydrogène, un radical alkyle en $C_1$ à $C_{10}$ ou un radical aryle ou aralkyle substitué ou non sur le noyau aromatique, les radicaux $R^2$, $R^3$ et/ou $R^4$ pouvant être reliés ou non à un polymère de type polystyrène ou polyacrylique, et dans lequel le(s) groupe(s) fonctionnel(s) présent(s) et à protéger dans la ou les chaîne(s) latérale(s) de l'acide aminé ou du peptide est (sont) bloqué (s) par le groupe Y de formule $R^1$-CH=CH-$CH_2$-, $R^1$ représentant un atome d'hydrogène ou un groupe alkyle en $C_1$ à $C_4$, sous forme de carbonates pour les groupes fonctionnels -OH alcool, de thiocarbonates ou de thiométhylènecarbamates pour les groupes fonctionnels -SH, d'éthers ou de carbonates pour les groupes fonctionnels -OH phénolique, de carbamates pour les groupes fonctionnels amino ou imino et d'esters pour les groupes fonctionnels -COOH,
   - on forme éventuellement une dernière liaison peptitique en condensant le peptide précédemment obtenu avec un dérivé d'acide aminé ou d'un peptide dans lequel la fonction amine en α est protégée si nécessaire par un groupe protecteur habituel et dans lequel le(s) groupe(s) fonctionnel(s) présent(s) et à protéger dans la ou les chaîne(s) latérale(s) de l'acide aminé ou du peptide est(sont) bloqué(s) par le groupe Y comme indiqué précédemment,
   - on élimine si nécessaire le groupe protecteur de la fonction amine en α éventuellement présent et/ou les groupes protecteurs Y des groupes fonctionnels des chaînes latérales et/ou le groupe protecteur de la fonction acide en α ; les groupes protecteurs de la fonction amine en α, autres que de type Y, et de la fonction acide en α étant enlevés de façon connue, les groupes protecteurs Y étant enlevés tous en même temps par une catalyse douce au moyen d'un composé d'un métal noble tel qu'un métal du groupe du platine, ruthénium, rhodium, osmium, iridium, platine ou palladium.

2. Procédé selon la revendication 1, caractérisé en ce que le groupe Y qui protège les groupes fonctionnels des chaînes latérales est le groupe de formule $CH_2$=CH-$CH_2$-.

3. Procédé selon la revendication 1 ou 2, caractérisé en ce que la fonction amine en α du premier dérivé d'acide aminé ou du peptide à allonger est protégée par le groupe Boc ou Fmoc.

4. Procédé selon la revendication 1 ou 2, caractérisé en ce que la fonction acide en α du second dérivé d'acide aminé ou du peptide à allonger est protégée sous forme d'ester par un radical alkyle en $C_1$ à $C_5$ ou benzyle.

5. Procédé selon la revendication 1, caractérisé en ce qu'on enlève en premier le groupe protecteur de la fonction amine en α s'il n'est pas de type allylique, puis on enlève en même temps tous les groupes protecteurs de type allylique des groupes fonctionnels des chaînes latérales et ensuite s'il n'a pas été déjà éliminé au cours d'un des traitements précédents on enlève le groupe protecteur de la fonction acide en α.

**6.** Procédé selon la revendication 1 ou 5, caractérisé en ce que le groupe protecteur de la fonction amine en α du peptide est éliminé au moyen d'un acide fort lorsque la fonction amine est protégée par le groupe Boc ou par une amine secondaire dans un solvant lorsque la fonction amine est protégée par le groupe Fmoc.

**7.** Procédé selon la revendication 1 ou 6, caractérisé en ce que les radicaux Y des groupes fonctionnels des chaînes latérales du peptide sont éliminés au moyen d'une quantité catalytique d'un composé du rhodium (I) ou d'un composé du palladium (O) ou (II) dans un solvant en présence d'accepteurs nucléophiles ou de donneurs de protons.

**8.** Procédé selon la revendication 7, caractérisé en ce que les radicaux Y sont éliminés en faisant réagir le peptide avec l'hydrure de tributylétain en présence d'une quantité catalytique de palladium (O) tétrakis (triphénylphosphine) ou de palladium (II) dichlorobis(triphénylphosphine) et d'un composé faiblement acide tel que l'acide acétique, le p-nitrophénol ou l'acétate de pyridinium ou d'eau, dans un milieu solvant ou bien en faisant réagir le peptide avec une quantité catalytique de palladium (O) tétrakis(triphénylphosphine) en présence de N,N-diméthylsilylamine et d'acétate de triméthylsilyle dans un milieu solvant à bas point d'ébullition et aprotique et en traitant ensuite le composé obtenu avec du méthanol.

**9.** Dérivés d'acides aminés utiles pour la préparation de peptides caractérisés en ce qu'ils ont la formule générale

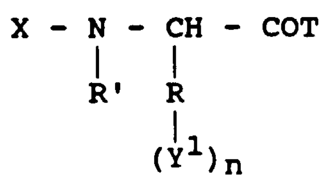

(I)

dans laquelle

X représente le groupe tert-butyloxycarbonyle (Boc), 9-fluorénylméthyloxycarbonyle (Fmoc), méthoxybenzyloxycarbonyle (Moz), α,α-diméthyl-3,5-diméthoxybenzyloxycarbonyle (Ddz) ou 2-nitrophénylsulfényle (Nps),
T représente le groupe OH, le radical $-OR^2$ dans lequel $R^2$ représente un radical alkyle en $C_1$ à $C_{10}$, un radical aryle ou aralkyle substitué ou non sur le noyau aromatique ou le radical phénacyle ou T représente le radical $-NR^3R^4$ dans lequel $R^3$ et $R^4$ identiques ou différents représentent un atome d'hydrogène, un radical alkyle en $C_1$ à $C_{10}$ ou un radical aryle ou aralkyle substitué ou non sur le noyau aromatique,
R' représente un atome d'hydrogène et R représente le reste de la chaîne latérale d'un acide aminé qui contient au moins un groupe fonctionnel -OH,-SH, >NH, =NH, $-NH_2$, -COOH ou bien R' et R sont liés et forment le reste d'un acide aminé cyclique qui porte au moins l'un des groupes fonctionnels précédents,
$Y^1$ représente le radical

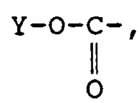

dans lequel Y a pour formule $R^1-CH=CH-CH_2-$, $R^1$ représentant un atome d'hydrogène ou un groupe alkyle en $C_1$ à $C_4$, lorsque $Y^1$ remplace l'atome d'hydrogène du ou des groupe(s) fonctionnel(s) -OH, -SH, >NH, =NH, ou $-NH_2$ contenu(s) dans le reste R ou dans le reste formé par R lié à R', sauf lorsque X est le radical tert-butyloxycarbonyle, T représente le groupe -OH et R représente le reste de la chaîne latérale de la lysine, ou $Y^1$ représente le groupe

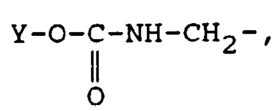

Y ayant la signification indiquée ci-dessus, lorsque $Y^1$ remplace l'atome d'hydrogène du ou des groupe(s) fonctionnel(s) -SH contenu(s) dans le reste R ou le reste formé par R lié à R',
ou bien $Y^1$ représente le radical Y de formule $R^1-CH=CH-CH_2-$, $R^1$ ayant les significations indiquées ci-dessus,

lorsque $Y^1$ remplace l'atome d'hydrogène du ou des groupe(s) fonctionnel(s) -COOH et/ou -OH phénolique contenu(s) dans le reste R ou le reste formé par R lié à R', sauf lorsque X est le radical tert-butyloxycarbonyle, T représente le groupe -OH et R représente le reste de la chaîne latérale de l'acide aspartique, de l'acide glutamique ou de la tyrosine ou T représente le groupe $-OR^2$ dans lequel $R^2$ est un radical alkyle et R représente le reste de la chaîne latérale de la tyrosine ou dans lequel $R^2$ est un radical alkyle ou aralkyle et R représente le reste de la chaîne latérale de l'acide glutamique;

n = 1 lorsque R ne contient qu'un seul groupe fonctionnel ou n = 1 ou 2 lorsque R contient deux groupes fonctionnels.

**10.** Dérivés selon la revendication précédente, caractérisés en ce que Y représente le radical $CH_2=CH-CH_2-$.

**11.** Dérivés selon la revendication 9 ou 10, caractérisés en ce que T représente le groupe -OH ou un radical $-OR^2$ dans lequel $R^2$ est un radical alkyle en $C_1$ à $C_5$ ou le radical benzyle et X représente le groupe Boc ou Fmoc.

**12.** Procédé de préparation des dérivés d'acides aminés de formule (I) caractérisé en ce que lorsque $Y^1$ représente le radical

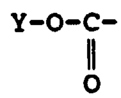

dans lequel Y a pour formule $R^1-CH=CH-CH_2-$, $R^1$ représentant un atome d'hydrogène ou un groupe alkyle en $C_1$ à $C_4$, on fait réagir un chloroformiate de formule

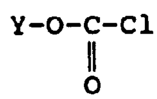

sur le composé de formule

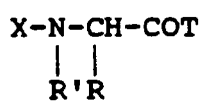

dans lesquelles Y, X, T, R et R' ont les significations précédentes, en milieu anhydre ou en milieu aqueux neutre ou basique selon le(s) groupe(s) fonctionnel(s) présent(s) dans le reste R ou le reste formé par R et R', à une température comprise entre 0° et 50°C.

**13.** Procédé de préparation selon la revendication précédente, caractérisé en ce que lorsque le groupe fonctionnel latéral à protéger est un groupe amino ou imino, la réaction a lieu dans un milieu aqueux à un pH compris entre 7 et 12.

**14.** Procédé de préparation selon la revendication 12, caractérisé en ce que lorsque R représente le reste de la sérine la réaction a lieu en milieu aqueux neutre.

**15.** Procédé de préparation selon la revendication 12, caractérisé en ce que lorsque R représente le reste de la thréonine on fait réagir en milieu anhydre le chloroformiate

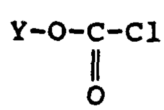

sur le composé de formule

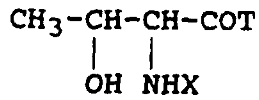

dans laquelle X a les significations précédentes et T représente le radical $-OR^2$ ou $-NR^3R^4$, $R^2$, $R^3$, $R^4$ ayant les significations précédentes, en présence de diméthylaminopyridine.

**16.** Procédé de préparation des dérivés d'acides aminés de formule (I) caractérisé en ce que lorsque $Y^1$ représente le radical Y de formule $R^1$-CH=CH-$CH_2$-, $R^1$ représentant un atome d'hydrogène ou un groupe alkyle en $C_1$ à $C_4$, on fait réagir un composé de formule Y-Br, Y ayant la signification précédente, en milieu basique sur le complexe cuivrique de l'acide aminé, éventuellement dans un solvant miscible à l'eau, à une température comprise entre 0° et 50°C puis on introduit le groupe protecteur X après élimination du cuivre et éventuellement on transforme la fonction -COOH en α, en ester $-COOR^2$ ou en amide $-CONR^3R^4$, $R^2$, $R^3$ et $R^4$ ayant les significations précédentes.

**17.** Procédé de préparation des dérivés d'acides aminés de formule (I) caractérisé en ce que lorsque $Y^1$ représente le groupe

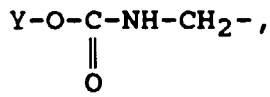

Y ayant la signification précédemment indiquée, on fait réagir l'hydroxyméthylcarbamate du groupe de type allyle sur l'acide aminé ou son chlorhydrate en milieu acide à une température comprise entre 0° et 50°, éventuellement dans un solvant aprotique puis on introduit le groupe protecteur X et éventuellement on transforme la fonction -COOH en α comme précédemment.

## Patentansprüche

**1.** Verfahren zur Herstellung von Peptiden in Lösung oder durch Festphasensynthese, gekennzeichnet durch folgende Schritte:

- Bildung einer Peptidbindung durch Kondensation eines ersten Derivats einer Aminosäure oder eines zu verlängernden Peptids, bei denen die α-Aminogruppe mit einer Gruppe geschützt ist, die unter tert.-Butyloxycarbonyl (Boc), 9-Fluorenylmethyloxycarbonyl (Fmoc), Methoxybenzyloxycarbonyl (Moz), α,α-Dimethyl-3,5-dimethoxybenzyloxycarbonyl (Ddz) und 2-Nitrophenylsulfenyl (Nps) ausgewählt ist, und worin die vorliegende (n) und zu schützende(n) funktionelle(n) Gruppe(n) in der oder den Seitenketten der Aminosäure oder des Peptids mit einer Gruppe Y der Formel $R^1$-CH=CH-$CH_2$- blockiert ist (sind), wobei $R^1$ ein Wasserstoffatom oder eine $C_{1-4}$-Alkylgruppe bedeutet, in Form von Carbonaten, wenn es sich um alkoholische funktionelle OH-Gruppen handelt, von Thiocarbonaten oder Thiomethylencarbamaten, wenn es sich um funktionelle Gruppen -SH handelt, von Ethern oder Carbonaten, wenn es sich um phenolische funktionelle OH-Gruppen handelt, von Carbamaten, wenn es sich um funktionelle Amino- oder Iminogruppen handelt, und von Estern, wenn es sich um funktionelle Gruppen -COOH handelt, mit einem zweiten Derivat einer Aminosäure oder eines zu verlängernden Peptids, bei denen die Säuregruppe -COOH in α-Stellung in Form einer Estergruppe $-COOR^2$, wobei $R^2$ eine $C_{1-10}$-Alkylgruppe, eine Arylgruppe oder eine Aralkylgruppe, die gegebenenfalls am aromatischen Ring substituiert sind, oder Phenacyl bedeutet, oder in Form einer Amidgruppe $-CONR^3R^4$ geschützt ist, wobei $R^3$ und $R^4$, die identisch oder voneinander verschieden sind, ein Wasserstoffatom, eine $C_{1-10}$-Alkylgruppe oder eine Arylgruppe oder eine Aralkylgruppe bedeutet, die gegebenenfalls am aromatischen Ring substituiert sind, wobei die Gruppen $R^2$, $R^3$ und/oder $R^4$ gegebenenfalls an ein Polymer vom Polystyroltyp oder Polyacryltyp gebunden sind, und bei denen die vorliegende(n) und zu schützende(n) funktionelle(n) Gruppe(n) an der oder den Seitenketten der Aminosäure oder des Peptids mit einer Gruppe Y der Formel $R^1$-CH=CH-$CH_2$- blockiert ist (sind), wobei $R^1$ ein Wasserstoffatom oder eine $C_{1-4}$-Alkylgruppe bedeutet, in Form von Carbonaten für funktionelle alkoholische OH-Gruppen, vonThiocarbonaten oder Thiomethylencarbamaten für funktionelle Gruppen -SH, von Ethern oder Carbonaten für phenolische funktionelle OH-Gruppen, von Carbamaten für funktionelle Amino- oder Iminogruppen und von Estern für funktionelle Gruppen -COOH,

- gegebenenfalls Bildung einer letzten Peptidbindung durch Kondensation des zuvor erhaltenen Peptids mit einem Derivat einer Aminosäure oder eines Peptids, bei denen die α-Aminogruppe erforderlichenfalls mit einer üblichen Schutzgruppe geschützt ist und die vorliegende(n) und zu schützende(n) funktionelle(n) Gruppe(n) in der oder den Seitenketten der Aminosäure oder des Peptids mit der obengenannten Gruppe Y blokkiert ist (sind),
- erforderlichenfalls Eliminierung der gegebenenfalls vorliegenden Schutzgruppe der α-Aminogruppe und/oder der Schutzgruppen Y der funktionellen Gruppen in den Seitenketten und/oder der Schutzgruppe der α-Säuregruppe, wobei die Schutzgruppen der α-Aminogruppe, die nicht vom Typ Y sind, und der α-Säuregruppe durch bekannte Verfahren abgespalten werden und wobei alle Schutzgruppen Y durch eine milde Katalyse mit einer Verbindung eines Edelmetalls, wie eines Metalls aus der Gruppe von Platin, Ruthenium, Rhodium, Osmium, Iridium, Platin oder Palladium, gleichzeitig abgespalten werden.

**2.** Verfahren nach Anspruch 1, dadurch gekennzeichnet, daß die Gruppe Y, die die funktionellen Gruppen der Seitenketten schützt, eine Gruppe der Formel $CH_2=CH-CH_2-$ ist.

**3.** Verfahren nach Anspruch 1 oder 2, dadurch gekennzeichnet, daß die α-Aminogruppe des ersten Derivats der Aminosäure oder des zu verlängernden Peptids durch die Gruppe Boc oder Fmoc geschützt ist.

**4.** Verfahren nach Anspruch 1 oder 2, dadurch gekennzeichnet, daß die α-Säuregruppe des zweiten Derivats der Aminosäure oder des zu verlängernden Peptids in Form eines Esters mit einer $C_{1-5}$-Alkylgruppe oder einer Benzylgruppe geschützt ist.

**5.** Verfahren nach Anspruch 1, dadurch gekennzeichnet, daß zunächst die Schutzgruppe der α-Aminogruppe, falls sie nicht vom Allyltyp ist, abgespalten wird, anschließend gleichzeitig alle Schutzgruppen der funktionellen Gruppen der Seitenketten vom Allyltyp abgespalten werden und dann, die Schutzgruppe der α-Säuregruppe abgespalten wird, falls sie nicht bereits im Laufe der vorhergehenden Behandlungen entfernt wurde.

**6.** Verfahren nach Anspruch 1 oder 5, dadurch gekennzeichnet, daß die Schutzgruppen der α-Aminogruppen des Peptids mit einer starken Säure abgespalten werden, wenn die Aminogruppen mit Boc-Gruppen geschützt sind, oder mit einem sekundären Amin in einem Lösungsmittel, wenn die Aminogruppen mit der Gruppe Fmoc geschützt sind.

**7.** Verfahren nach Anspruch 1 oder 6, dadurch gekennzeichnet, daß die Gruppen Y der funktionellen Gruppen der Seitenketten des Peptids mit einer katalytischen Menge einer Rhodium(I)-Verbindung oder einer Palladium(0)- oder Palladium(II)-Verbindung in einem Lösungsmittel in Gegenwart von nucleophilen Akzeptoren oder Protonendonatoren abgespalten wird.

**8.** Verfahren nach Anspruch 7, dadurch gekennzeichnet, daß die Gruppen Y entfernt werden, indem das Peptid mit Tributylzinnhydrid in Gegenwart einer katalytischen Menge Palladium(0)-Tetrakis(triphenylphosphin) oder Palladium(II)-Dichlorbis(triphenylphosphin) und einer schwach sauren Verbindung, wie Essigsäure oder p-Nitrophenol, oder von Pyridiniumacetat oder Wasser in einem Lösungsmittelmedium umgesetzt wird, oder indem das Peptid mit einer katalytischen Menge Palladium(0)-Tetrakis(triphenylphosphin) in Gegenwart von N,N-Dimethylsilylamin und Trimethylsilylacetat in einem aprotischen Lösungsmittelmedium mit niedrigem Siedepunkt umgesetzt und anschließend die erhaltene Verbindung mit Methanol behandelt wird.

**9.** Derivate von Aminosäuren, die zur Herstellung von Peptiden geeignet sind, dadurch gekennzeichnet, daß sie die allgemeine Formel

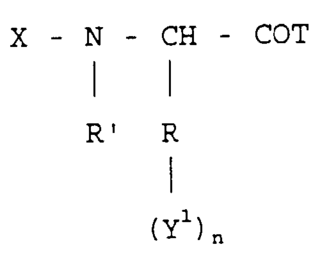

(I)

aufweisen, worin bedeuten:

X *tert.*-Butyloxycarbonyl (Boc), 9-Fluorenylmethyloxycarbonyl (Fmoc), Methoxybenzyloxycarbonyl (Moz), $\alpha$, $\alpha$-Dimethyl-3,5-dimethoxybenzyloxycarbonyl (Ddz) oder 2-Nitrophenylsulfenyl (Nps),
T -OH, -$OR^2$, wobei $R^2$ eine $C_{1-10}$-Alkylgruppe, eine Arylgruppe oder eine Aralkylgruppe, die gegebenenfalls am aromatischen Ring substituiert sind, oder Phenacyl bedeutet, oder -$NR^3R^4$, wobei $R^3$ und $R^4$, die identisch oder voneinander verschieden sind, ein Wasserstoffatom, eine $C_{1-10}$-Alkylgruppe oder eine Arylgruppe oder eine Aralkylgruppe bedeuten, die gegebenenfalls am aromatischen Ring substituiert sind,
R' Wasserstoff und
R den Rest der Seitenkette einer Aminosäure, die mindestens eine der funktionellen Gruppen -OH, -SH, $>$NH, =NH, -$NH_2$ und -COOH enthält, oder
R' und R miteinander verbunden den Rest einer cyclischen Aminosäure, die mindestens eine der vorgenannten funktionellen Gruppen aufweist,
$Y^1$ die Gruppe

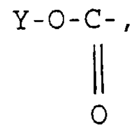

wobei Y die Formel $R^1$-CH=CH-$CH_2$- aufweist, wobei $R^1$ ein Wasserstoffatom oder eine $C_{1-4}$-Alkylgruppe bedeutet, wenn $Y^1$ das Wasserstoffatom der funktionellen Gruppe(n) -OH, -SH, $>$NH, =NH, oder -$NH_2$ ersetzt, die in dem Rest R oder in dem Rest, der durch die Verbindung von R mit R' gebildet wird, enthalten ist (sind), außer, wenn X die Gruppe *tert.*-Butyloxycarbonyl, T die Gruppe -OH und R den Rest der Seitenkette von Lysin bedeuten, oder
$Y^1$ die Gruppe

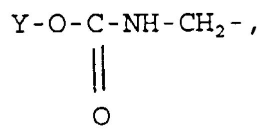

wobei Y die oben angegebene Bedeutung aufweist, wenn $Y^1$ das Wasserstoffatom der funktionelle(n) Gruppe (n) -SH ersetzt, die im Rest R oder dem Rest, der durch die Verbindung von R mit R' gebildet wird, enthalten ist (sind), oder
$Y^1$ die Gruppe Y der Formel $R^1$-CH=CH-$CH_2$-, wobei $R^1$ die oben angegebenen Bedeutungen aufweist, wenn $Y^1$ das Wasserstoffatom der funktionellen Gruppe(n) -COOH und/oder phenolisches -OH ersetzt, die im Rest R oder dem Rest, der durch die Verbindung von R mit R' gebildet wird, enthalten ist (sind), außer, wenn X die Gruppe tert.- Butyloxycarbonyl, T die Gruppe -OH und R den Rest der Seitenkette von Asparaginsäure, Glutaminsäure oder Tyrosin oder T die Gruppe -$OR^2$, wobei $R^2$ eine Alkylgruppe ist und R den Rest der Seitenkette von Tyrosin bedeuten oder $R^2$ eine Alkylgruppe oder Aralkylgruppe ist und R den Rest der Seitenkette von Glutaminsäure bedeutet, und
n = 1, wenn R nur eine einzige funktionelle Gruppe enthält oder n = 1 oder 2, wenn R zwei funktionelle Gruppen enthält.

**10.** Derivate nach dem vorhergehenden Anspruch, dadurch gekennzeichnet, daß Y die Gruppe $CH_2$=CH-$CH_2$- bedeutet.

**11.** Derivate nach Anspruch 9 oder 10, dadurch gekennzeichnet, daß T die Gruppe -OH oder eine Gruppe -$OR^2$ bedeutet, wobei $R^2$ eine $C_{1-5}$-Alkylgruppe oder eine Benzylgruppe ist, und X die Gruppe Boc oder Fmoc bedeutet.

**12.** Verfahren zur Herstellung der Aminosäurederivate der Formel (I), dadurch gekennzeichnet, daß, wenn $Y^1$ die Gruppe

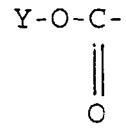

bedeutet, worin Y die Formel $R^1$-CH=CH-$CH_2$- aufweist, in der $R^1$ Wasserstoff oder eine $C_{1-4}$-Alkylgruppe ist, ein Chlorformiat der Formel

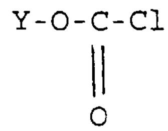

mit einer Verbindung der Formel

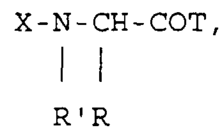

wobei Y, X, T, R und R' die oben angegebenen Bedeutungen aufweisen, in wasserfreiem Medium oder in einem neutralen oder basischen wäßrigen Medium je nach der Art der in dem Rest R oder in dem Rest, der durch R und R' gebildet wird, vorliegenden funktionellen Gruppe(n) bei einer Temperatur von 0 bis 50 °C umgesetzt wird.

**13.** Verfahren nach dem vorhergehenden Anspruch, dadurch gekennzeichnet, daß, wenn die funktionelle zu schützende Seitengruppe eine Amino- oder Iminogruppe ist, die Reaktion in einem wäßrigen Medium bei einem pH-Wert von 7 bis 12 durchgeführt wird.

**14.** Verfahren nach Anspruch 12, dadurch gekennzeichnet, daß, wenn R der Serinrest ist, die Reaktion in neutralem wäßrigem Medium durchgeführt wird.

**15.** Verfahren nach Anspruch 12, dadurch gekennzeichnet, daß, wenn R den Threoninrest bedeutet, das Chlorformiat

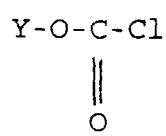

in wasserfreiem Medium mit der Verbindung der Formel

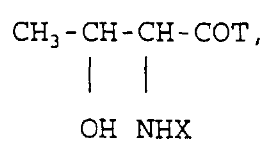

worin X die oben angegebenen Bedeutungen aufweist und T die Gruppe -$OR^2$ oder -$NR^3R^4$ ist, wobei $R^2$, $R^3$ und $R^4$ die oben angegebenen Bedeutungen haben, in Gegenwart von Dimethylaminopyridin umgesetzt wird.

**16.** Verfahren zur Herstellung der Aminosäurederivate der Formel (I), dadurch gekennzeichnet, daß, wenn $Y^1$ die Gruppe Y der Formel $R^1$-CH=CH-$CH_2$- ist, wobei $R^1$ Wasserstoff oder eine $C_{1-4}$-Alkylgruppe bedeutet, eine Verbindung der Formel Y-Br, wobei Y die obengenannte Bedeutung hat, in basischem Medium mit einem Kupferkomplex der Aminosäure, gegebenenfalls in einem mit Wasser mischbaren Lösungsmittel, bei einer Temperatur im

Bereich von 0 bis 50 °C umgesetzt wird, worauf die Schutzgruppe X nach Entfernen des Kupfers zugegeben wird und gegebenenfalls die Gruppen -COOH in α-Stellung in einen Ester $-COOR^2$ oder ein Amid $-CONR^3R^4$ umgewandelt werden, wobei $R^2$, $R^3$ und $R^4$ die obengenannten Bedeutungen aufweisen.

**17.** Verfahren zur Herstellung der Aminosäurederivate der Formel (I), dadurch gekennzeichnet, daß, wenn $Y^1$ die Gruppe

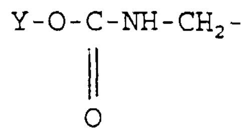

bedeutet, wobei Y die obengenannte Bedeutung hat, das Hydroxymethylcarbamat der Gruppe vom Allyltyp mit einer Aminosäure oder ihrem Hydrochlorid in saurem Medium bei einer Temperatur von 0 bis 50 °C gegebenenfalls in einem aprotischen Lösungsmittel, umgesetzt wird, worauf die Schutzgruppe X eingeführt wird und gegebenenfalls die Gruppen -COOH in α-Stellung wie vorher umgewandelt werden.

## Claims

**1.** A process for preparing peptides in solution or in the solid phase, characterised in that:

- the peptide link is formed by condensing a first derivative of an amino acid or of a peptide to be extended in which the amino function in the α-position is protected by a group selected from tert-butyloxycarbonyl (Boc), 9-fluorenylmethyloxycarbonyl (Fmoc), methoxybenyloxycarbonyl (Moz), α,α-dimethyl-3,5-dimethoxybenzyloxycarbonyl (Ddz) and 2-nitrophenylsulphenyl (Nps), and in which functional group(s) which are present and to be protected in the side chain(s) of the amino acid or the peptide is/are blocked by group Y of the formula $R^1-CH=CH-CH_2-$, where $R^1$ represents a hydrogen atom or a $C_1$-$C_4$-alkyl group, in the form of carbonates when these are alcoholic -OH functional groups, thiocarbonates or thiomethylenecarbamates when these are -SH functional groups, ethers or carbonates when these are phenolic -OH functional groups, carbamates when these are amino or imino functional groups and esters when these are -COOH functional groups, with a second amino acid derivative or a peptide to be extended in which the -COOH acid function in the α-position is protected in the form of an ester group $-COOR^2$ in which $R^2$ is a $C_1$-$C_{10}$-alkyl radical, an aryl or aralkyl radical, substituted or not in the aromatic ring or the phenacyl radical, or in the form of an amide group $-CONR^3R^4$ in which $R^3$ and $R^4$ are identical or different and represent a hydrogen atom, a $C_1$-$C_{10}$-alkyl radical or an aryl or aralkyl radical, substituted or not in the aromatic ring, the radicals $R^2$, $R^3$ and/or $R^4$ being able to be linked or not to a polymer of the polystyrene or polyacrylic type, and in which the functional groups present and to be protected in the side chain(s) of the amino acid or peptide is/are blocked by group Y of the formula $R^1-CH=CH-CH_2-$, where $R^1$ represents a hydrogen atom or a $C_1$-$C_4$-alkyl group, in the form of carbonates for alcoholic -OH functional groups, thiocarbonates or thiomethylenecarbamates for -SH functional groups, ethers or carbonates for phenolic -OH groups, carbamates for amino or imino functional groups and esters for -COOH functional groups,
- a final peptide link is optionally formed by condensing the peptide obtained previously with an amino acid derivative or a peptide in which the amine function in the α-position is protected, if necessary, by a conventional protective group and in which the functional groups(s) present and to be protected in the side chain(s) of the amino acid or the peptide is/are blocked by group Y as indicated above,
- if necessary, the protective group for the amine function group in the α-position which may be present, and/or the protective groups Y for the functional groups in the side chains and/or the protective group for the acid function in the α-position are removed; protective groups for the amine function in the α-position, other than of the type Y, and for the acid function in the α-position being removed in a known manner, all the protective groups Y being removed at the same time by mild catalysis using a compound of a noble metal such as a metal from the platinum group, ruthenium, rhodium, osmium, iridium, platinum or palladium.

**2.** A process according to Claim 1, characterised in that group Y which protects functional groups in side chains is the group of the formula $CH_2=CH-CH_2-$.

**3.** A process according to Claim 1 or 2, characterised in that the amine function in the α-position in the first amino

acid derivative or peptide to be extended is protected by the group Boc or Fmoc.

**4.** A process according to Claim 1 or 2, characterised in that the acid function in the α-position in the second amino acid derivative or peptide to be extended is protected in the form of an ester by a $C_1$-$C_5$-alkyl or benzyl radical.

**5.** A process according to Claim 1, characterised in that the protective group on the amine function in the α-position is removed first, if it is not of the allyl type, then all the protective groups of the allyl type are removed at the same time from the functional groups in the side chains and then, if it has not already been removed during the course of the preceding reactions, the protective group is removed from the acid function in the α-position.

**6.** A process according to Claim 1 or 5, characterised in that the protective group for the amine function in the α-position in the peptide is eliminated using a strong acid when the amine function is protected by the Boc group or by a secondary amine in a solvent when the amine function is protected by the Fmoc group.

**7.** A process according to Claim 1 or 6, characterised in that the Y radicals on the functional groups in the side chains of the peptide are eliminated using a catalytic amount of a rhodium (I) compound or a palladium (0) or (II) compound in a solvent in the presence of nucleophilic acceptors or proton donors.

**8.** A process according to Claim 7, characterised in that the Y radicals are eliminated by reacting the peptide with tributyltin hydride in the presence of a catalytic amount of palladium (0) tetrakis(triphenylphosphine) or of palladium (II) dichlorobis (triphenylphosphine) and a weakly acidic compound such as acetic acid, p-nitrophenol or pyridinium acetate or water, in a solvent medium or else by reacting the peptide with a catalytic amount of palladium (0) tetrakis(triphenylphosphine) in the presence of N,N-dimethylsilylamine and trimethylsilyl acetate in a low-boiling and aprotic solvent medium and by then treating the compound obtained with methanol.

**9.** Amino acid derivatives which are suitable for preparing peptides characterised in that they have the general formula

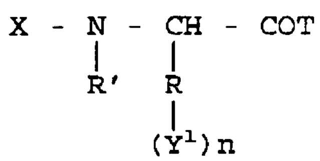

(I)

in which

X represents the group tert-butyloxycarbonyl (Boc), 9-fluorenylmethyloxycarbonyl (Fmoc), methoxybenzyloxycarbonyl (Moz), α,α-dimethyl-3,5-dimethoxybenzyloxycarbonyl (Ddz) or 2-nitrophenylsulphenyl (Nps),
T represents the group OH, the radical $-OR^2$ in which $R^2$ represents a $C_1$-$C_{10}$-alkyl radical, an aryl or alkaryl radical, substituted or not in the aromatic ring, or the phenacyl radical or T represents the $-NR^3R^4$ radical in which $R^3$ and $R^4$ are identical or different and represent a hydrogen atom, a $C_1$-$C_{10}$-alkyl radical or an aryl or aralkyl radical, substituted or not in the aromatic ring,
R' represents a hydrogen atom and R represents the grouping from the side chain of an amino acid which contains at least one functional group -OH, -SH, >NH =NH, $-NH_2$, -COOH or else R' and R are linked and form the grouping from a cyclic amino acid which carries at least one of the preceding functional groups,
$Y^1$ represents the radical

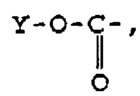

in which Y has the formula $R^1$-CH=CH-$CH_2$-, $R^1$ representing a hydrogen atom or a $C_1$-$C_4$-alkyl group, when $Y^1$ replaces the hydrogen atom in the functional groups(s) -OH, -SH, >NH, =NH or $-NH_2$ contained in the grouping R or in the grouping formed by R linked to R', except when X is the tert-butyloxycarbonyl radical, T represents the group -OH and R represents the grouping from the side chain in lysine,
or $Y^1$ represents the group

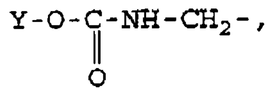

Y being defined in the same way as above, when $Y^1$ replaces the hydrogen atom in -SH functional group(s) contained in the grouping R or in the grouping formed by R linked to R', or else $Y^1$ represents the radical Y of the formula $R^1$-CH=CH-$CH_2$-, $R^1$ being defined in the same way as above, when $Y^1$ replaces the hydrogen atom in -COOH and/or phenolic -OH functional groups contained in the grouping R or the grouping formed by R linked to R', except when X is the tert-butyloxycarbonyl radical, T represents the -OH group and R represents the grouping from the side chain in aspartic acid, glutamic acid or tyrosine or T represents the -$OR^2$ group in which $R^2$ is an alkyl radical and R represents the grouping from the side chain of tyrosine or in which $R^2$ is an alkyl or aralkyl radical and R represents the grouping from the side chain of glutamic acid;
n = 1 when R contains only one functional group or n = 1 or 2 when R contains two functional groups.

**10.** Derivatives according to the preceding Claim, characterised in that Y represents the $CH_2$=CH-$CH_2$- radical.

**11.** Derivatives according to Claims 9 or 10, characterised in that T represents the -OH group or an -$OR^2$ radical in which $R^2$ is a $C_1$-$C_5$-alkyl radical or the benzyl radical and X represents the group Boc or Fmoc.

**12.** A method for preparing amino acid derivatives of the formula (I), characterised in that when $Y^1$ represents the radical

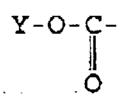

in which Y has the formula $R^1$-CH=CH-$CH_2$-, $R^1$ representing a hydrogen atom or a $C_1$-$C_4$-alkyl radical, a chloroformate of the formula

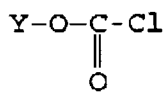

is reacted with a compound of the formula

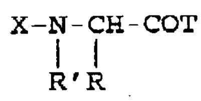

in which Y, X, T, R and R' are defined in the same way as above, in an anhydrous medium or in a neutral or basic aqueous medium, depending on the functional groups(s) which are present in the grouping R or the grouping formed by R and R', at a temperature between 0°C and 50°C.

**13.** A method of preparation according to the preceding Claim, characterised in that when the side functional group to be protected is an amino or imino group, reaction takes place in an aqueous medium at a pH between 7 and 12.

**14.** A method of preparation according to Claim 12, characterised in that when R represents the serine grouping, reaction takes place in neutral aqueous medium.

**15.** A method of preparation according to Claim 12, characterised in that when R represents the threonine grouping, the chloroformate

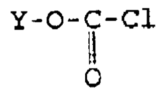

is reacted in an anhydrous medium with the compound of the formula

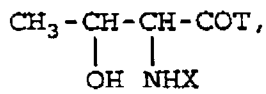

in which X is defined in the same way as above and T represents the $-OR^2$ or $-NR^3R^4$ radical, $R^2$, $R^3$ and $R^4$ being defined in the same way as above, in the presence of dimethylaminopyridine.

**16.** A method for preparing amino acid derivatives of the formula (I), characterised in that when $Y^1$ represents the radical Y of the formula $R^1-CH=CH-CH_2-$, $R^1$ representing a hydrogen atom or a $C_1$-$C_4$-alkyl radical, a compound of the formula Y-Br, Y being defined in the same way as above, is reacted in a basic medium with a copper complex of the amino acid, optionally in a solvent which is miscible with water, at a temperature between 0°C and 50°C, then the protective group X is introduced after elimination of the copper and optionally the -COOH group in the α-position is converted into an ester $-COOR^2$ or an amide $-CONR^3R^4$, $R^2$, $R^3$ and $R^4$ being defined in the same way as above.

**17.** A method for preparing amino acid derivatives of the formula (I), characterised in that when $Y^1$ represents the group

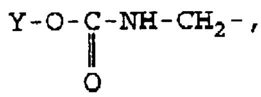

Y being defined in the same way as above, the hydroxymethylcarbamate from the allyl type group is reacted with the amino acid or its chlorohydrate in an acid medium at a temperature between 0°C and 50°C, optionally in an aprotic solvent, then the protective group X is introduced and optionally the -COOH function in the α-position is converted in the same way as described above.